# EUROPÄISCHE PATENTANMELDUNG

(11) **EP 3 991 573 A1**
(43) Veröffentlichungstag der Anmeldung: **04.05.2022**
(21) Anmeldenummer: 20205430.0
(22) Anmeldetag: 03.11.2020
(51) Int. Cl.: A23L 33/175, A61K 9/00, A61K 31/198, A61K 31/405

(54) **ZUSAMMENSETZUNGEN ZUR NASALEN APPLIKATION**

(71) Anmelder: Faber, Patrick, 1230 Wien (AT)
(72) Erfinder: Faber, Patrick, 1230 Wien (AT)
(74) Vertreter: SONN Patentanwälte OG

(57) **Zusammenfassung**

Die Erfindung betrifft pulverförmige Zusammensetzungen zur nasalen Applikation enthaltend Taurin, Koffein, L-Arginin und L-Tryptophan. Die Erfindung betrifft weiters Behältnisse und Sets enthaltend derartige Zusammensetzungen sowie Verwendungen dieser Zusammensetzungen.

## Beschreibung

Das Gebiet der vorliegenden Erfindung sind Zusammensetzungen enthaltend Taurin und Koffein sowie deren Verwendungen.

Einnehmbare Zusammensetzungen enthaltend Koffein und/oder Taurin sind aus dem Stand der Technik bekannt. Häufig handelt es sich dabei um Getränkezubereitungen, z.B. "Energy-Drinks", welche zusätzlich Vitamine, Zucker oder Süßstoffe enthalten können. Die Einnahme solcher Energy Drinks kann eine vorübergehende leistungssteigernde Wirkung haben.

Beispielsweise offenbart die WO 2006/084894 A2 Nahrungsmittel und Getränke, welche Koffein, Taurin und Tryptophan enthalten. Die Verabreichung von Tryptophan zusammen mit Koffein und Taurin soll dabei zu einer wesentlichen Verbesserung der kognitiven Leistungsfähigkeit führen, indem die neurale Effizienz gesteigert wird.

Trotz zahlreicher aus dem Stand der Technik bekannten Zusammensetzungen besteht weiterhin ein Bedarf an verbesserten Zusammensetzungen enthaltend Taurin und/oder Koffein. Insbesondere besteht ein Bedarf an Zusammensetzungen, welche eine verbesserte Wirkung und/oder bessere Verträglichkeit aufweisen.

Es ist eine Aufgabe der vorliegenden Erfindung alternative Zusammensetzungen enthaltend Taurin und Koffein, insbesondere Zusammensetzungen mit verbesserter Wirkung und/oder besserer Verträglichkeit, zur Verfügung zu stellen.

Die vorliegende Erfindung stellt daher eine pulverförmige Zusammensetzung zur nasalen Applikation enthaltend Taurin, Koffein, L-Arginin und L-Tryptophan zur Verfügung.

Ein weiterer Aspekt der vorliegenden Erfindung betrifft ein Behältnis enthaltend eine erfindungsgemäße pulverförmige Zusammensetzung. Zusätzlich stellt die Erfindung ein Set enthaltend eine pulverförmige Zusammensetzung gemäß der Erfindung und einen nasalen Applikator (wie einen Schnupflöffel oder ein Schnupfröhrchen) zur Verfügung. Darüber hinaus betrifft die Erfindung die Verwendung einer pulverförmigen Zusammensetzung, eines Behältnisses, oder eines Sets gemäß der Erfindung zur nasalen Applikation der pulverförmigen Zusammensetzung.

Weiters betrifft die Erfindung unterschiedliche Verwendungen von pulverförmigen Zusammensetzungen enthaltend Taurin, Koffein, L-Arginin und L-Tryptophan, unter anderem zur zur Verbesserung der kognitiven Leistungsfähigkeit, insbesondere zur Steigerung der Konzentrationsfähigkeit und/oder der neuralen Effizienz, zur Verhinderung einer Überaktivierung von Hirnregionen bei Stressbelastung und/oder zur Verbesserung der Downregulierung nach einer Stressbelastung, zur Verbesserung der körperlichen Leistungsfähigkeit, insbesondere zur Steigerung der Kraft und/oder der Ausdauer, zur Stimmungsaufhellung, zur Anregung der Gehirnaktivität, als Aroma, und/oder zur Verstärkung der Wirkung von Aromen, wobei die pulverförmige Zusammensetzung nasal appliziert wird, vorzugsweise wobei es sich bei der Zusammensetzung um die erfindungsgemäße pulverförmige Zusammensetzung handelt.

Im Zusammenhang mit der vorliegenden Erfindung wurde ein überraschendes Zusammenwirken, einerseits zwischen den einzelnen Inhaltsstoffen der erfindungsgemäßen Zusammensetzung, und andererseits zwischen diesen Inhaltsstoffen und einer nasalen Verabreichung beobachtet. Aus der WO 2006/084894 A2 war zwar bereits bekannt, dass die Verabreichung von Tryptophan zusammen mit Koffein und Taurin zu einer Verbesserung der kognitiven Leistungsfähigkeit führen kann. Im Rahmen der vorliegenden Erfindung hat sich nun einerseits herausgestellt, dass auch Arginin in Kombination mit Taurin und Koffein vorteilhafte Effekte haben kann und andererseits, dass Arginin mit den anderen Inhaltsstoffen zusammenwirken und den in der WO 2006/08484 A2 beschriebenen Effekt von Tryptophan noch verstärken kann. So hat sich gezeigt, dass Arginin sowohl eine Verstärkung der Wirkung bei selber Dosis, als auch eine schneller einsetzende bzw. länger anhaltende Wirkung ermöglichen kann. Darüber hinaus hat sich gezeigt, dass die nasale Verabreichung der Zusammensetzung, z.B. als Schnupfpulver, zu Vorteilen in Bezug auf Stärke und Verlauf der Wirkung sowie auf die Verträglichkeit führt, insbesondere im Zusammenwirken mit Arginin in der Zusammensetzung. Die erfindungsgemäße Kombination von Inhaltsstoffen sowie die nasale Verabreichung ermöglichen außerdem eine Reihe neuer Verwendungen.

Bei der erfindungsgemäßen Zusammensetzung handelt es sich vorzugsweise um ein Schnupfpulver, d.h. die Zusammensetzung ist vorzugsweise zum Schnupfen geeignet. Bei Schnupfpulver handelt es sich üblicherweise um fein pulverisierte Zusammensetzungen, welche durch stoßweises, kräftiges Einatmen in die Nasenlöcher eingezogen werden können. Vorzugsweise ist die Zusammensetzung zur Verabreichung an einen Menschen, insbesondere als Schnupfpulver für einen Menschen, geeignet.

Es hat sich gezeigt, dass es vorteilhaft ist, wenn die Partikel der pulverförmigen Zusammensetzung nicht zu fein sind. Ohne an eine bestimmte Theorie gebunden zu sein, wird vermutet, dass bei einer nasalen Applikation kleinere Partikel eher in die Lunge gelangen, während größere Partikel direkt über die Nasenschleimhaut aufgenommen werden und sich so die vorteilhaften Wirkungen der Erfindung besser entfalten.

Es ist daher bevorzugt, dass die Zusammensetzung eine mittlere Partikelgröße von mehr als 1 µm, bevorzugt mehr als 5 µm, mehr bevorzugt mehr als 10 µm, noch mehr bevorzugt mehr als 20 µm, am meisten bevorzugt mehr als 50 µm aufweist. In nach Präferenz aufsteigender Reihenfolge ist es bevorzugt, wenn die Zusammensetzung eine mittlere Partikelgröße von mehr als 1, 2, 3, 4, 5, 6, 7, 8, 9, 10, 11, 12, 13, 14, 15, 16, 17, 18, 19, 20, 25, 30, 35, 40, 45, 50 µm aufweist.

Um ein einfaches Einziehen durch die Nase sowie eine effiziente Aufnahme zu gewährleisten, ist es andererseits bevorzugt, dass die Zusammensetzung eine mittlere Partikelgröße von weniger als 1.000 µm, bevorzugt weniger als 500 µm, mehr bevorzugt weniger als 200 µm, noch mehr bevorzugt weniger als 50 µm, am meisten bevorzugt weniger als 20 µm aufweist. Besonders bevorzugt ist es, dass die Zusammensetzung eine mittlere Partikelgröße zwischen 1 µm und 1.000 µm, bevorzugt zwischen 5 µm und 500 µm, noch mehr bevorzugt zwischen 20 µm und 200 µm, am meisten bevorzugt zwischen 50 µm und 100 µm aufweist.

Wie hierin verwendet bezeichnet die mittlere Partikelgröße den D50-Wert der Partikelgrößenverteilung. Der D50-Wert bezeichnet den Wert, unter welchem sich 50% der Partikelgrößenverteilung befinden. Analog dazu können auch ein D10- und ein D90-Wert bestimmt werden, als die Werte, unter denen sich 10% bzw. 90% der Partikelgrößenverteilung befinden. Die Spanne ist ein Maß für die Breite der Partikelgrößenverteilung und kann bestimmt werden als Spanne = (D90 - D10) / D50.

Im Zusammenhang mit der vorliegenden Erfindung wird die Partikelgrößenverteilung, insbesondere die D50-, D10, und D90-Werte, vorzugsweise durch Laserbeugungs-Partikelgrößenanalyse bestimmt. Dabei entspricht die Partikelgröße vorzugsweise dem Äquivalentdurchmesser der beugungsgleichen Kugel. Die mittlere Partikelgröße entspricht vorzugsweise der volumenbasierten mittlere Partikelgröße, insbesondere dem D50-Wert einer volumetrischen Partikelgrößenverteilung. Vorzugsweise wird die Laserbeutungs-Partikelgrößenanalyse gemäß der Norm ISO 13320:2020 durchgeführt. Beispielsweise kann die mittlere Partikelgröße mit dem Gerät Mastersizer 3000 der Firma Malvern Panalytical bestimmt werden.

Alternativ dazu kann die mittlere Partikelgröße auch durch Siebanalyse bestimmt werden. Der Fachmann ist mit der Durchführung einer solchen Analyse vertraut. Vorzugsweise wird die Siebanalyse durchgeführt gemäß der Deutschen Norm DIN 66165-1:2016 08.

Im Rahmen der Erfindung ist es bevorzugt, wenn der Anteil sehr feiner Partikel gering ist, unter anderem, da sehr feine Partikel bei der nasalen Applikation vermehrt in die Lunge gelangen können anstatt über die Nasenschleimhaut aufgenommen zu werden. Es ist daher bevorzugt, dass die Zusammensetzung eine Partikelgrößenverteilung mit einem D10-Wert von mindestens 100 nm, vorzugsweise mindestens 500 nm, mehr bevorzugt mindestens 1 µm, am meisten bevorzugt mindestens 2 µm aufweist. Vorzugsweise beträgt der D10-Wert zwischen 100 nm und 50 µm, bevorzugt zwischen 500 nm und 20 µm, noch mehr bevorzugt zwischen 1 µm und 15 µm, am meisten bevorzugt zwischen 2 µm und 10 µm.

Es ist weiters bevorzugt, dass die erfindungsgemäße pulverförmige Zusammensetzung eine Partikelgrößenverteilung mit einer niedrigen Spanne ((D90 - D10) / D50) aufweist. Insbesondere ist die Spanne kleiner als 500, bevorzugt kleiner als 100, noch mehr bevorzugt kleiner als 20, am meisten bevorzugt kleiner als 10.

Vorzugsweise enthält die erfindungsgemäße pulverförmige Zusammensetzung mindestens 1 Gew.-%, bevorzugt mindestens 2 Gew.-%, mehr bevorzugt mindestens 4 Gew.-%, noch mehr bevorzugt mindestens 8 Gew.-%, noch mehr bevorzugt mindestens 15 Gew.-%, am meisten bevorzugt mindestens 20 Gew.-% Taurin. Gereiht nach aufsteigender Präferenz enthält die Zusammensetzung vorzugsweise mindestens 1, 2, 3, 4, 5, 6, 7, 8, 9, 10, 11, 12, 13, 14, 15, 16, 17, 18, 19, 20 Gew.-% Taurin. Besonders bevorzugt ist es, dass die Zusammensetzung zwischen 1 Gew.-% und 75 Gew.-%, bevorzugt zwischen 2 Gew.-% und 60 Gew.-%, mehr bevorzugt zwischen 4 Gew.-% und 50 Gew.-%, noch mehr bevorzugt zwischen 8 Gew.-% und 40 Gew.-%, noch mehr bevorzugt zwischen 15 Gew.-% und 35 Gew.-%, am meisten bevorzugt zwischen 20 Gew.-% und 30 Gew.-% Taurin enthält.

Vorzugsweise enthält die erfindungsgemäße pulverförmige Zusammensetzung mindestens 0,25 Gew.-%, bevorzugt mindestens 0,5 Gew.-%, mehr bevorzugt mindestens 1 Gew.-%, noch mehr bevorzugt mindestens 2 Gew.-%, noch mehr bevorzugt mindestens 4 Gew.-%, am meisten bevorzugt mindestens 8 Gew.-% Koffein. Gereiht nach aufsteigender Präferenz enthält die Zusammensetzung vorzugsweise mindestens 0,25, 0,5, 0,75, 1, 1,25, 1,5, 1,75, 2, 2,25, 2,5, 2,75, 3, 3,25, 3,5, 3,75, 4, 4,25, 4,5, 4,75, 5, 5,25, 5,5, 5,75, 6, 6,25, 6,5, 6,75, 7, 7,25, 7,5, 7,75, 8 Gew.-% Koffein. Besonders bevorzugt ist es, dass die Zusammensetzung zwischen 0,25 Gew.-% und 50 Gew.-%, bevorzugt zwischen 0.5 Gew.-% und 35 Gew.-%, mehr bevorzugt zwischen 1 Gew.-% und 25 Gew.-%, noch mehr bevorzugt zwischen 2 Gew.-% und 20 Gew.-%, noch mehr bevorzugt zwischen 4 Gew.-% und 15 Gew.-%, am meisten bevorzugt zwischen 8 Gew.-% und 12 Gew.-% Koffein enthält.

Vorzugsweise enthält die erfindungsgemäße pulverförmige Zusammensetzung mindestens 0,5 Gew.-%, bevorzugt mindestens 1 Gew.-%, mehr bevorzugt mindestens 2 Gew.-%, noch mehr bevorzugt mindestens 4 Gew.-%, noch mehr bevorzugt mindestens 8 Gew.-%, am meisten bevorzugt mindestens 14 Gew.-% L-Arginin. Gereiht nach aufsteigender Präferenz enthält die Zusammensetzung vorzugsweise mindestens 0,5, 1, 1,5, 2, 2,5, 3, 3,5, 4, 4,5, 5, 5,5, 6, 6,5, 7, 7,5, 8, 8,5, 9, 9,5, 10, 10,5, 11, 11,5, 12, 12,5, 13, 13,5, 14 Gew.-% L-Arginin. Besonders bevorzugt ist es, dass die Zusammensetzung zwischen 0,5 Gew.-% und 50 Gew.-%, bevorzugt zwischen 1 Gew.-% und 40 Gew.-%, mehr bevorzugt zwischen 2 Gew.-% und 35 Gew.-%, noch mehr bevorzugt zwischen 4 Gew.-% und 28 Gew.-%, noch mehr bevorzugt zwischen 8 Gew.-% und 22 Gew.-%, am meisten bevorzugt zwischen 14 Gew.-% und 19 Gew.-% L-Arginin enthält.

Vorzugsweise enthält die erfindungsgemäße pulverförmige Zusammensetzung mindestens 0,25 Gew.-%, bevorzugt mindestens 0,5 Gew.-%, mehr bevorzugt mindestens 1 Gew.-%, noch mehr bevorzugt mindestens 2 Gew.-%, noch mehr bevorzugt mindestens 4 Gew.-%, am meisten bevorzugt mindestens 8 Gew.-% L-Tryptophan. Gereiht nach aufsteigender Präferenz enthält die Zusammensetzung vorzugsweise mindestens 0,25, 0,5, 0,75, 1, 1,25, 1,5, 1,75, 2, 2,25, 2,5, 2,75, 3, 3,25, 3,5, 3,75, 4, 4,25, 4,5, 4, 75, 5, 5,25, 5,5, 5,75, 6, 6,25, 6,5, 6,75, 7, 7,25, 7,5, 7,75, 8 Gew.-% L-Tryptophan. Besonders bevorzugt ist es, dass die Zusammensetzung zwischen 0,25 Gew.-% und 50 Gew.-%, bevorzugt zwischen 0.5 Gew.-% und 35 Gew.-%, mehr bevorzugt zwischen 1 Gew.-% und 25 Gew.-%, noch mehr bevorzugt zwischen 2 Gew.-% und 20 Gew.-%, noch mehr bevorzugt zwischen 4 Gew.-% und 15 Gew.-%, am meisten bevorzugt zwischen 8 Gew.-% und 12 Gew.-% L-Tryptophan enthält.

In einer bevorzugten Ausführungsform enthält die erfindungsgemäße pulverförmige Zusammensetzung Füllstoffe, vorzugsweise Maltodextrin.

In einer weiteren bevorzugten Ausführungsform enthält die Zusammensetzung zusätzlich Aromastoffe, vorzugsweise Menthol, Vanillin, oder Fruchtaromen. Die Möglichkeit, unterschiedliche Aromastoffe in der Zusammensetzung vorzusehen, erlaubt es, unterschiedliche Erlebnisse bei der Anwendung zu erzeugen. Beispielsweise können aktivierende oder auch beruhigende und wohltuende Aromen gewählt werden.

In einer weiteren bevorzugten Ausführungsform enthält die Zusammensetzung zusätzlich einen fluoreszierenden Farbstoff, vorzugsweise einen fluoreszierenden Lebensmittelfarbstoff, insbesondere Riboflavin, Tartrazin, Gelborange S, Cochenillerot A, Amaranth, oder Brillantblau FCF. Vorzugsweise lässt sich der fluoreszierende Farbstoff durch UV-Licht, vorzugsweise mit Licht einer Wellenlänge zwischen 200 nm und 400 nm, insbesondere 365 nm, anregen. Durch Vorsehen eines fluoreszierenden Farbstoffs ist die pulverförmige Zusammensetzung auch in nur mit Schwarzlicht (UV-Licht) beleuchteten Räumen gut sichtbar, was eine besonders einfache Anwendung in solchen Umgebungen ermöglicht.

In einer besonders bevorzugten Ausführungsform enthält die Zusammensetzung:
- zwischen 4 Gew.-% und 40 Gew.-% Taurin;
- zwischen 2 Gew.-% und 25 Gew.-% Koffein;
- zwischen 1 Gew.-% und 17 Gew.-% L-Arginin; und
- zwischen 1 Gew.-% und 17 Gew.-% L-Tryptophan.

In einer weiteren besonders bevorzugten Ausführungsform besteht die Zusammensetzung aus:
- zwischen 4 Gew.-% und 40 Gew.-% Taurin;
- zwischen 2 Gew.-% und 25 Gew.-% Koffein;
- zwischen 1 Gew.-% und 17 Gew.-% L-Arginin;
- zwischen 1 Gew.-% und 17 Gew.-% L-Tryptophan; und
- ad 100 Gew.-% Füllstoffe, insbesondere Maltodextrin.

Da die erfindungsgemäßen pulverförmigen Zusammensetzungen zur nasalen Applikation vorgesehen sind, ist es bevorzugt, dass gewisse Inhaltsstoffe, welche sich häufig in Nahrungsmitteln bzw. Energy-Drinks wiederfinden, nicht oder nur in geringer Konzentration enthalten sind. Derartige Inhaltsstoffe können unter anderem die Nasenschleimhaut reizen oder zu anderen unerwünschten Effekt führen.

Es ist daher bevorzugt, dass die erfindungsgemäße Zusammensetzung weniger als 10 Gew.-%, bevorzugt weniger als 5 Gew.-%, noch mehr bevorzugt weniger als 1 Gew.-%, noch mehr bevorzugt weniger als 0,1 Gew.-%, am meisten bevorzugt keine Süßungsmittel enthält. Süßungsmittel, wie hierin verwendet, werden als Zuckerersatzstoffe verstanden, d.h. Lebensmittelzusatzstoffe, die Lebensmitteln einen süßen Geschmack geben sollen. Zucker, wie beispielsweise Maltodextrin, sind keine Süßungsmittel im Sinne der Erfindung. In einer bevorzugten Ausführungsform sind die Süßungsmittel Süßstoffe, vorzugsweise ausgewählt aus der Gruppe bestehend aus Acesulfam, Advantam, Aspartam, Cyclamat, Neohesperidin, Neotam, Saccharin, Sucralose, Steviosid und Thaumatin. In einer weiteren bevorzugten Ausführungsform sind die Süßungsmittel Zuckeraustauschstoffe, vorzugsweise Zuckeralkohole, insbesondere wobei die Zuckeraustauschstoffe ausgewählt sind aus der Gruppe bestehend aus Sorbit, Mannit, Isomalt, Maltit, Polyglycitol, Laktit, Xylit und Erythrit.

Darüber hinaus ist es bevorzugt, dass die Zusammensetzung weniger als 10 Gew.-%, bevorzugt weniger als 5 Gew.-%, noch mehr bevorzugt weniger als 1 Gew.-%, noch mehr bevorzugt weniger als 0,1 Gew.-%, am meisten bevorzugt keine Citronensäure oder Citrate enthält. Des weiteren ist es bevorzugt, dass die Zusammensetzung weniger als 10 Gew.-%, bevorzugt weniger als 5 Gew.-%, noch mehr bevorzugt weniger als 1 Gew.-%, noch mehr bevorzugt weniger als 0,1 Gew.-%, noch mehr bevorzugt weniger als 0,01 Gew.-%, am meisten bevorzugt kein Nikotin enthält.

In einem weiteren Aspekt betrifft die Erfindung ein Behältnis enthaltend die pulverförmige Zusammensetzung gemäß der Erfindung. Alle beschriebenen bevorzugten Ausführungsformen der erfindungsgemäßen Zusammensetzung gelten ebenso als bevorzugte Ausführungsformen für das erfindungsgemäße Behältnis.

In einer bevorzugten Ausführungsform enthält das Behältnis mindestens 10 mg, bevorzugt mindestens 50 mg, mehr bevorzugt mindestens 200 mg, noch mehr bevorzugt mindestens 400 mg, am meisten bevorzugt mindestens 800 mg der pulverförmigen Zusammensetzung. Insbesondere bevorzugt ist es, wenn das Behältnis zwischen 10 mg und 20.000 mg, bevorzugt zwischen 50 mg und 10.000 mg, mehr bevorzugt zwischen 200 mg und 5.000 mg, noch mehr bevorzugt zwischen 400 mg und 2.500 mg, am meisten bevorzugt zwischen 800 mg und 1.250 mg der pulverförmigen Zusammensetzung enthält. Im Rahmen der Erfindung hat sich herausgestellt, dass sich diese Menge besonders gut für eine einzelne Anwendung bzw. für mehrere aufeinanderfolgende Anwendungen eignet. Das Behältnis enthält somit vorzugsweise eine vorportionierte Menge der erfindungsgemäßen Zusammensetzung für eine Anwendung und kann nach dem Konsum einfach entsorgt werden. Dadurch wird sichergestellt, dass der Anwender eine optimale Menge der Zusammensetzung konsumiert, ohne dass er diese Menge durch wägen oder Ähnliches sicherstellen muss.

Jegliche Art von Behältnis kann im Rahmen der Erfindung verwendet werden. Vorzugsweise handelt es sich dabei um ein Sachet, eine Dose, eine Ampulle, ein Glas oder eine Flasche. Insbesondere Sachets haben sich als besonders praktikabel erwiesen, da diese einfach transportiert werden können und nach Konsum der darin enthaltenen Zusammensetzung einfach entsorgt werden können.

In einem weiteren Aspekt betrifft die Erfindung ein Set enthaltend eine pulverförmige Zusammensetzung gemäß der Erfindung und einen nasalen Applikator wie einen Schnupflöffel oder ein Schnupfröhrchen. Vorzugsweise enthält das Set ein erfindungsgemäßes Behältnis und einen nasalen Applikator wie einen Schnupflöffel oder ein Schnupfröhrchen. Alle beschriebenen bevorzugten Ausführungsformen der erfindungsgemäßen Zusammensetzung und des erfindungsgemäßen Behältnisses gelten ebenso als bevorzugte Ausführungsformen für das erfindungsgemäße Set.

Ein Schnupfröhrchen, oft auch als Nasenröhrchen oder Ziehröhrchen bezeichnet, ist ein Gegenstand, welcher den nasalen Konsum von pulverförmigen Zusammensetzungen erleichtert. Das Schnupfröhrchen kann am Nasenloch angesetzt werden und die Zusammensetzung kann durch stoßweises kräftiges Einatmen durch das Schnupfröhrchen in die Nase gezogen werden. Vorzugsweise ist das Schnupfröchrchen rohrförmig oder rinnenförmig ausgestaltet.

Ein weiterer nasaler Applikator ist der Schnupflöffel - ein löffelförmiger Gegenstand, welcher die nasale Anwendung von pulverförmigen Zusammensetzungen erleichtert. Daneben ermöglicht der Schnupflöffel die Entnahme einer Dosis der pulverförmigen Zusammensetzung aus dem Behältnis zur nasalen Anwendung.

In einer bevorzugten Ausführungsform enthält das Set mindestens zwei, vorzugsweise mindestens drei, insbesondere mindestens fünf Behältnisse gemäß der Erfindung. Jedes dieser Behältnisse kann wiederum eine vorportionierte Menge der erfindungsgemäßen Zusammensetzung enthalten, sodass der Konsument bei jeder Anwendung eine optimale Menge der Zusammensetzung zu sich nehmen kann, ohne diese abwägen zu müssen.

In einem weiteren Aspekt betrifft die vorliegende Erfindung eine Verwendung einer pulverförmige Zusammensetzung, einem Behältnis, oder einem Set gemäß der Erfindung zur nasalen Applikation der pulverförmigen Zusammensetzung. Die Applikation kann beispielsweise durch Einziehen in die Nase der auf einer Oberfläche verteilten Zusammensetzung, unmittelbar aus dem erfindungsgemäßen Behältnis (z.B. aus einer Dose), und/oder über einen nasalen Applikator wie einen Schnupflöffel oder ein Schnupfröhrchen erfolgen.

Vorzugsweise ist die Verwendung zur nasalen Applikation der pulverförmigen Zusammensetzung an einem Menschen. Die Verwendung erfolgt daher vorzugsweise durch einen Menschen. Vorzugsweise handelt es sich bei dem Menschen um einen neurologisch und psychiatrisch gesunde Person bzw. um eine Person, welche keine neurologischen und/oder psychiatrischen Erkrankungen aufweist. Insbesondere handelt es sich um einen gesunden Menschen.

In einer bevorzugten Ausführungsform umfasst die Verwendung die nasale Applikation von mindestens 10 mg, bevorzugt mindestens 50 mg, mehr bevorzugt mindestens 200 mg, noch mehr bevorzugt mindestens 400 mg, am meisten bevorzugt mindestens 800 mg der pulverförmigen Zusammensetzung. Insbesondere ist es bevorzugt, dass die Verwendung die nasale Applikation von zwischen 10 mg und 5.000 mg, bevorzugt zwischen 50 mg und 4.000 mg, mehr bevorzugt zwischen 200 mg und 3.000 mg, noch mehr bevorzugt zwischen 400 mg und 2.000 mg, am meisten bevorzugt zwischen 800 mg und 1.250 mg der pulverförmigen Zusammensetzung umfasst. Diese Mengen haben sich als besonders günstig für eine einzelne Anwendung, bzw. für mehrere aufeinanderfolgende Anwendungen, erwiesen.

In weiteren Aspekten, welche in der Folge genauer beschrieben werden, betrifft die Erfindung eine Reihe unterschiedliche Verwendungen pulverförmiger Zusammensetzungen, wobei die pulverförmige Zusammensetzung nasal appliziert wird. Im Zusammenhang mit allen beschriebenen Verwendungen ist die pulverförmiger Zusammensetzung vorzugsweise eine erfindungsgemäße pulverförmige Zusammensetzung wie hierin beschrieben. Für alle beschriebenen Verwendungen kann anstelle der pulverförmigen Zusammensetzung auch das erfindungsgemäße Behältnis oder das erfindungsgemäße Set eingesetzt werden (welche jeweils die erfindungsgemäße pulverförmige Zusammensetzung enthalten). Alle beschriebenen bevorzugten Ausführungsformen der erfindungsgemäßen Zusammensetzung, des erfindungsgemäßen Behältnisses, des erfindungsgemäßen Sets und der einzelnen erfindungsgemäßen Verwendungen gelten ebenso als bevorzugte Ausführungsformen für alle hierein offenbarten Verwendungen.

Bei den hierin genannten Verwendungen beträgt die Tagesdosis der pulverförmigen Zusammensetzung vorzugsweise zwischen 10 mg und 20.000 mg, bevorzugt zwischen 50 mg und 10.000 mg, mehr bevorzugt zwischen 200 mg und 5.000 mg, noch mehr bevorzugt zwischen 400 mg und 2.500 mg, am meisten bevorzugt zwischen 800 mg und 1.250 mg. Diese Tagesdosis kann in einer oder mehreren Anwendungen verabreicht werden, beispielsweise in 10 Anwendungen mit einer jeweiligen Dosis von 100 mg.

Ein Aspekt der Erfindung betrifft die Verwendung einer pulverförmigen Zusammensetzung enthaltend Taurin, Koffein, L-Arginin und L-Tryptophan zur Verbesserung der kognitiven Leistungsfähigkeit, insbesondere zur Steigerung der Konzentrationsfähigkeit und/oder der neuralen Effizienz, wobei die pulverförmige Zusammensetzung nasal appliziert wird.

Insbesondere in Bezug auf die Konzentrationsfähigkeit hat sich gezeigt, dass das Vorliegen von Arginin in der Zusammensetzung zu einer besonders starken Wirkung, unter anderem auch zu einer überraschenden Verlängerung der Wirkung führt. Weiters hat sich gezeigt, dass die nasale Verabreichung einer Aufnahme über die Nahrung, beispielsweise als Getränkt, überlegen ist.

Das Phänomen der sogenannten neuralen Effizienz ist beschrieben z.B. in Grabner, et al. ("When intelligence loses its impact: Neural efficiency during reasoning in a familiar area." International Journal of Psychophysiology 49.2 (2003): 89-98), Neubauer und Fink ("Intelligence and neural efficiency." Neuroscience & Biobehavioral Reviews 33.7 (2009): 1004-1023) und Dunst, et al. ("Neural efficiency as a function of task demands." Intelligence 42 (2014): 22-30). Bildgebende Elektronenzephalogramm (EEG) - Verfahren haben gezeigt, dass intelligente Menschen die Aktivierung des Gehirns auf kleinere Areale der Großhirnrinde beschränken können, und zwar vor allem auf solche, die für die Bearbeitung der jeweiligen Aufgabe wirklich notwendig sind. Weniger Begabte hingegen müssen größere Teile ihres Gehirns aktivieren und verbrauchen so für dieselbe Aufgabe mehr Ressourcen. Die neurale Effizienz ist ein Maß für diese psychophysiologischen Unterschiede, die bei Denkprozessen intelligenter und weniger intelligenter Menschen auftreten. Das Phänomen der selektiven Aktivierung von Gehirnbereichen, welches bei besonders intelligenten Menschen auftritt, wird auch als "Genieeffekt" bezeichnet.

Aus der WO 2006/084894 A1 war bereits bekannt, dass die orale Verabreichung von Tryptophan zusammen mit Koffein und Taurin zu einer Steigerung der neuralen Effizienz und zu einer Verbesserung der kognitiven Leistungsfähigkeit führen kann. Im Rahmen der vorliegenden Erfindung hat sich ergeben, dass einerseits die zusätzliche Kombination mit Arginin und andererseits eine nasale Verabreichung eine Verbesserung dieser Effekte ermöglicht.

Zur Erbringung einer bestimmten kognitiven und konzentrativen Leistung werden nach Verabreichung der erfindungsgemäßen Zusammensetzung die zur Erbringung der betreffenden Leistung erforderlichen Hirnregionen zielgerichteter aktiviert. Insbesondere erfolgt eine Zentrierung der Aktivitätserhöhung auf diejenigen Hirnrindenregionen, die zur Bewältigung einer bestimmten kognitiven Aufgabe erforderlich sind. Von besonderem Vorteil ist ferner, dass der sich an eine Stressbeanspruchung anschließende Erholungsprozess auf ein stärker reduziertes Niveau führt, das heißt der Erholungszeitraum wird verkürzt. Die erfindungsgemäße Zusammensetzung führt also zu dem "Genieeffekt", d.h. einer Gehirnaktivität hoher neuraler Effizienz, wie sie sonst nur bei besonders intelligenten Menschen vorkommt. Dieser Effekt ist gegenüber den aus der WO 2006/084894 A1 bekannten oral zu verabreichenden Zusammensetzungen in Bezug auf die Stärke und Verlauf (Einsetzen und Dauer) der Wirkung noch verbessert.

Ein weiterer Aspekt der Erfindung betrifft die Verwendung einer pulverförmigen Zusammensetzung enthaltend Taurin, Koffein, L-Arginin und L-Tryptophan zur Verhinderung einer Überaktivierung von Hirnregionen bei Stressbelastung und/oder zur Verbesserung der Downregulierung nach einer Stressbelastung, wobei die pulverförmige Zusammensetzung nasal appliziert wird. Die erfindungsgemäße Zusammensetzung kann überschießende Stressreaktionen dämpfen, während jedoch die hormonellen Reaktionen, die zur Mobilisierung der ergotropen Ressourcen notwendig ist, erhalten werden. Die down-Regulation von der zentral-nervöse Aktivierung kann beschleunigt werden.

Im Rahmen der Erfindung hat sich gezeigt, dass die erfindungsgemäße Zusammensetzung, insbesondere durch das Vorliegen von L-Arginin und die nasale Anwendung, besonders vorteilhafte Effekte auf die körperliche Leistungsfähigkeit hat. Insbesondere wird eine starke, schnell einsetzende und lang andauernde Wirkung ermöglicht. Ein weiterer Aspekt der Erfindung betrifft daher die Verwendung einer pulverförmigen Zusammensetzung enthaltend Taurin, Koffein, L-Arginin und L-Tryptophan zur Verbesserung der körperlichen Leistungsfähigkeit, insbesondere zur Steigerung der Kraft und/oder der Ausdauer, wobei die pulverförmige Zusammensetzung nasal appliziert wird.

Darüber hinaus kann die erfindungsgemäße Zusammensetzung bei einem gesunden Menschen zu einer Serotoninausschüttung und Stimmungsaufhellung, sowie zu einer Anregung der Gehirnaktivität führen. Ein weiterer Aspekt der Erfindung betrifft daher die Verwendung einer pulverförmigen Zusammensetzung enthaltend Taurin, Koffein, L-Arginin und L-Tryptophan zur Stimmungsaufhellung, wobei die pulverförmige Zusammensetzung nasal appliziert wird. Die Erfindung betrifft zusätzlich die Verwendung einer pulverförmigen Zusammensetzung enthaltend Taurin, Koffein, L-Arginin und L-Tryptophan zur Anregung der Gehirnaktivität, wobei die pulverförmige Zusammensetzung nasal appliziert wird.

Überraschenderweise hat sich gezeigt, dass die erfindungsgemäße pulverförmige Zusammensetzung besonders vorteilhaft als Aroma eingesetzt werden kann. Nach einer nasalen Verabreichung führt einerseits die Zusammensetzung selbst zu einem positiven Aromaerlebnis, andererseits wirkt die Zusammensetzung auch als "Aromakatalysator" für andere Aromen. So werden nach Konsum der erfindungsgemäßen Zusammensetzung andere Aromen intensiver wahrgenommen. Die Wirkung von beispielsweise einem Raumaroma oder einem anderen verabreichten Aroma wird dadurch verstärkt. Ohne an eine bestimmte Theorie gebunden zu sein, wird vermutet, dass die nasale Applikation eine direkte Wirkung der Zusammensetzung auf das Riechepithel in der Nasenhöhle ermöglicht.

Ein weiterer Aspekt der Erfindung betrifft daher die Verwendung einer pulverförmigen Zusammensetzung enthaltend Taurin, Koffein, L-Arginin und L-Tryptophan als Aroma, wobei die pulverförmige Zusammensetzung nasal appliziert wird.

Die Erfindung betrifft daher weiters die Verwendung einer pulverförmigen Zusammensetzung enthaltend Taurin, Koffein, L-Arginin und L-Tryptophan zur Verstärkung der Wirkung von Aromen, wobei die pulverförmige Zusammensetzung nasal appliziert wird. Die Verwendung umfasst vorzugsweise die Verabreichung von zumindest eines weiteren Aromas. Das weitere Aroma kann ebenfalls nasal appliziert werden, z.B. als Pulver, als Flüssigkeit, oder als Aerosol, oder auch über die Raumluft, z.B. über einen Diffuser oder eine Duftkerze, verabreicht werden. Das weitere Aroma ist vorzugsweise ein synthetischer oder natürlicher Aromastoff, z.B. Vanillin oder Menthol, oder ein Aromaextrakt, insbesondere aus Früchten, Gewürzen, oder Kräutern, oder ein ätherisches Öl. Die Verwendung kann vorteilhafterweise auch vor der Einnahme von Speisen und/oder Getränken erfolgen, beispielsweise in Restaurants oder Bars, wodurch das Geschmackserlebnis intensiviert wird.

Sämtliche hierin genannten Parameter beziehen sich, wenn nicht anders gekennzeichnet, auf SATP-Bedingungen nach IUPAC ("Standard Ambient Temperature and Pressure"), insbesondere auf eine Temperatur von 25 °C und einen Druck von 101.300 Pa. Sämtliche Prozent-Angaben (%) hierin beziehen sich, wenn nicht anders gekennzeichnet, auf Gewichtsprozent. Sämtliche Angaben von Inhaltsstoffen der offenbarten Zusammensetzungen, insbesondere Taurin, Koffein, L-Arginin und L-Tryptophan, umfassen jeweils auch verträgliche Salze davon.

Die vorliegende Erfindung offenbart insbesondere die folgenden bevorzugten Ausführungsformen:
Ausführungsform 1: Pulverförmige Zusammensetzung zur nasalen Applikation enthaltend Taurin, Koffein, L-Arginin und L-Tryptophan.
Ausführungsform 2: Pulverförmige Zusammensetzung gemäß Ausführungsform 1, dadurch gekennzeichnet, dass die Zusammensetzung eine mittlere Partikelgröße von mehr als 1 µm, bevorzugt mehr als 5 µm, mehr bevorzugt mehr als 10 µm, noch mehr bevorzugt mehr als 20 µm, am meisten bevorzugt mehr als 50 µm aufweist.
Ausführungsform 3: Pulverförmige Zusammensetzung gemäß Ausführungsform 1 oder 2, dadurch gekennzeichnet, dass die Zusammensetzung eine mittlere Partikelgröße von weniger als 1.000 µm, bevorzugt weniger als 500 µm, mehr bevorzugt weniger als 200 µm, noch mehr bevorzugt weniger als 50 µm, am meisten bevorzugt weniger als 20 µm aufweist.
Ausführungsform 4: Pulverförmige Zusammensetzung gemäß einer der Ausführungsformen 1 bis 3, dadurch gekennzeichnet, dass die Zusammensetzung eine mittlere Partikelgröße zwischen 1 µm und 1.000 µm, bevorzugt zwischen 5 µm und 500 µm, noch mehr bevorzugt zwischen 20 µm und 200 µm, am meisten bevorzugt zwischen 50 µm und 100 µm aufweist.
Ausführungsform 5: Pulverförmige Zusammensetzung gemäß einer der Ausführungsformen 1 bis 4, dadurch gekennzeichnet, dass die Zusammensetzung mindestens 1 Gew.-%, bevorzugt mindestens 2 Gew.-%, mehr bevorzugt mindestens 4 Gew.-%, noch mehr bevorzugt mindestens 8 Gew.-%, noch mehr bevorzugt mindestens 15 Gew.-%, am meisten bevorzugt mindestens 20 Gew.-% Taurin enthält.
Ausführungsform 6: Pulverförmige Zusammensetzung gemäß einer der Ausführungsformen 1 bis 5, dadurch gekennzeichnet, dass die Zusammensetzung zwischen 1 Gew.-% und 75 Gew.-%, bevorzugt zwischen 2 Gew.-% und 60 Gew.-%, mehr bevorzugt zwischen 4 Gew.-% und 50 Gew.-%, noch mehr bevorzugt zwischen 8 Gew.-% und 40 Gew.-%, noch mehr bevorzugt zwischen 15 Gew.-% und 35 Gew.-%, am meisten bevorzugt zwischen 20 Gew.-% und 30 Gew.-% Taurin enthält.
Ausführungsform 7: Pulverförmige Zusammensetzung gemäß einer der Ausführungsformen 1 bis 6, dadurch gekennzeichnet, dass die Zusammensetzung mindestens 0,25 Gew.-%, bevorzugt mindestens 0,5 Gew.-%, mehr bevorzugt mindestens 1 Gew.-%, noch mehr bevorzugt mindestens 2 Gew.-%, noch mehr bevorzugt mindestens 4 Gew.-%, am meisten bevorzugt mindestens 8 Gew.-% Koffein enthält.
Ausführungsform 8: Pulverförmige Zusammensetzung gemäß einer der Ausführungsformen 1 bis 7, dadurch gekennzeichnet, dass die Zusammensetzung zwischen 0,25 Gew.-% und 50 Gew.-%, bevorzugt zwischen 0.5 Gew.-% und 35 Gew.-%, mehr bevorzugt zwischen 1 Gew.-% und 25 Gew.-%, noch mehr bevorzugt zwischen 2 Gew.-% und 20 Gew.-%, noch mehr bevorzugt zwischen 4 Gew.-% und 15 Gew.-%, am meisten bevorzugt zwischen 8 Gew.-% und 12 Gew.-% Koffein enthält.
Ausführungsform 9: Pulverförmige Zusammensetzung gemäß einer der Ausführungsformen 1 bis 8, dadurch gekennzeichnet, dass die Zusammensetzung mindestens 0,5 Gew.-%, bevorzugt mindestens 1 Gew.-%, mehr bevorzugt mindestens 2 Gew.-%, noch mehr bevorzugt mindestens 4 Gew.-%, noch mehr bevorzugt mindestens 8 Gew.-%, am meisten bevorzugt mindestens 14 Gew.-% L-Arginin enthält.
Ausführungsform 10: Pulverförmige Zusammensetzung gemäß einer der Ausführungsformen 1 bis 9, dadurch gekennzeichnet, dass die Zusammensetzung zwischen 0,5 Gew.-% und 50 Gew.-%, bevorzugt zwischen 1 Gew.-% und 40 Gew.-%, mehr bevorzugt zwischen 2 Gew.-% und 35 Gew.-%, noch mehr bevorzugt zwischen 4 Gew.-% und 28 Gew.-%, noch mehr bevorzugt zwischen 8 Gew.-% und 22 Gew.-%, am meisten bevorzugt zwischen 14 Gew.-% und 19 Gew.-% L-Arginin enthält.
Ausführungsform 11: Pulverförmige Zusammensetzung gemäß einer der Ausführungsformen 1 bis 10, dadurch gekennzeichnet, dass die Zusammensetzung mindestens 0,25 Gew.-%, bevorzugt mindestens 0,5 Gew.-%, mehr bevorzugt mindestens 1 Gew.-%, noch mehr bevorzugt mindestens 2 Gew.-%, noch mehr bevorzugt mindestens 4 Gew.-%, am meisten bevorzugt mindestens 8 Gew.-% L-Tryptophan enthält.
Ausführungsform 12: Pulverförmige Zusammensetzung gemäß einer der Ausführungsformen 1 bis 11, dadurch gekennzeichnet, dass die Zusammensetzung zwischen 0,25 Gew.-% und 50 Gew.-%, bevorzugt zwischen 0.5 Gew.-% und 35 Gew.-%, mehr bevorzugt zwischen 1 Gew.-% und 25 Gew.-%, noch mehr bevorzugt zwischen 2 Gew.-% und 20 Gew.-%, noch mehr bevorzugt zwischen 4 Gew.-% und 15 Gew.-%, am meisten bevorzugt zwischen 8 Gew.-% und 12 Gew.-% L-Tryptophan enthält.
Ausführungsform 13: Pulverförmige Zusammensetzung gemäß einer der Ausführungsformen 1 bis 12, dadurch gekennzeichnet, dass die Zusammensetzung zusätzlich Füllstoffe, vorzugsweise Maltodextrin, enthält.
Ausführungsform 14: Pulverförmige Zusammensetzung gemäß einer der Ausführungsformen 1 bis 13, dadurch gekennzeichnet, dass die Zusammensetzung zusätzlich Aromastoffe, vorzugsweise Menthol, Vanillin, oder Fruchtaromen, enthält.
Ausführungsform 15: Pulverförmige Zusammensetzung gemäß einer der Ausführungsformen 1 bis 14, dadurch gekennzeichnet, dass die Zusammensetzung zusätzlich einen fluoreszierenden Farbstoff, vorzugsweise einen fluoreszierenden Lebensmittelfarbstoff, insbesondere Riboflavin, Tartrazin, Gelborange S, Cochenillerot A, Amaranth, oder Brillantblau FCF, enthält.
Ausführungsform 16: Pulverförmige Zusammensetzung gemäß einer der Ausführungsformen 1 bis 15, dadurch gekennzeichnet, dass die Zusammensetzung enthält:
   - zwischen 4 Gew.-% und 40 Gew.-% Taurin;
   - zwischen 2 Gew.-% und 25 Gew.-% Koffein;
   - zwischen 1 Gew.-% und 17 Gew.-% L-Arginin; und
   - zwischen 1 Gew.-% und 17 Gew.-% L-Tryptophan.
Ausführungsform 17: Pulverförmige Zusammensetzung gemäß einer der Ausführungsformen 1 bis 13, dadurch gekennzeichnet, dass die Zusammensetzung besteht aus:
   - zwischen 4 Gew.-% und 40 Gew.-% Taurin;
   - zwischen 2 Gew.-% und 25 Gew.-% Koffein;
   - zwischen 1 Gew.-% und 17 Gew.-% L-Arginin;
   - zwischen 1 Gew.-% und 17 Gew.-% L-Tryptophan; und
   - ad 100 Gew.-% Füllstoffe.
Ausführungsform 18: Pulverförmige Zusammensetzung gemäß einer der Ausführungsformen 1 bis 17, dadurch gekennzeichnet, dass die Zusammensetzung weniger als 10 Gew.-%, bevorzugt weniger als 5 Gew.-%, noch mehr bevorzugt weniger als 1 Gew.-%, noch mehr bevorzugt weniger als 0,1 Gew.-%, am meisten bevorzugt keine Süßungsmittel enthält.
Ausführungsform 19: Pulverförmige Zusammensetzung gemäß Ausführungsform 18, dadurch gekennzeichnet, dass die Süßungsmittel Süßstoffe sind, vorzugsweise ausgewählt aus der Gruppe bestehend aus Acesulfam, Advantam, Aspartam, Cyclamat, Neohesperidin, Neotam, Saccharin, Sucralose, Steviosid und Thaumatin.
Ausführungsform 20: Pulverförmige Zusammensetzung gemäß Ausführungsform 18, dadurch gekennzeichnet, dass die Süßungsmittel Zuckeraustauschstoffe sind, vorzugsweise Zuckeralkohole, insbesondere wobei die Zuckeraustauschstoffe ausgewählt sind aus der Gruppe bestehend aus Sorbit, Mannit, Isomalt, Maltit, Polyglycitol, Laktit, Xylit und Erythrit.
Ausführungsform 21: Pulverförmige Zusammensetzung gemäß einer der Ausführungsformen 1 bis 20, dadurch gekennzeichnet, dass die Zusammensetzung weniger als 10 Gew.-%, bevorzugt weniger als 5 Gew.-%, noch mehr bevorzugt weniger als 1 Gew.-%, noch mehr bevorzugt weniger als 0,1 Gew.-%, am meisten bevorzugt keine Citronensäure oder Citrate enthält.
Ausführungsform 22: Pulverförmige Zusammensetzung gemäß einer der Ausführungsformen 1 bis 21, dadurch gekennzeichnet, dass die Zusammensetzung weniger als 10 Gew.-%, bevorzugt weniger als 5 Gew.-%, noch mehr bevorzugt weniger als 1 Gew.-%, noch mehr bevorzugt weniger als 0,1 Gew.-%, noch mehr bevorzugt weniger als 0,01 Gew.-%, am meisten bevorzugt kein Nikotin enthält.
Ausführungsform 23: Behältnis enthaltend eine pulverförmige Zusammensetzung gemäß einer der Ausführungsformen 1 bis 22.
Ausführungsform 24: Behältnis gemäß Ausführungsform 23, dadurch gekennzeichnet, dass das Behältnis mindestens 10 mg, bevorzugt mindestens 50 mg, mehr bevorzugt mindestens 200 mg, noch mehr bevorzugt mindestens 400 mg, am meisten bevorzugt mindestens 800 mg der pulverförmigen Zusammensetzung enthält.
Ausführungsform 25: Behältnis gemäß Ausführungsform 23 oder 24, dadurch gekennzeichnet, dass das Behältnis zwischen 10 mg und 20.000 mg, bevorzugt zwischen 50 mg und 10.000 mg, mehr bevorzugt zwischen 200 mg und 5.000 mg, noch mehr bevorzugt zwischen 400 mg und 2.500 mg, am meisten bevorzugt zwischen 800 mg und 1.250 mg der pulverförmigen Zusammensetzung enthält.
Ausführungsform 26: Behältnis gemäß einer der Ausführungsformen 23 bis 25, dadurch gekennzeichnet, dass das Behältnis ein Sachet, eine Dose, eine Ampulle, ein Glas oder eine Flasche ist.
Ausführungsform 27: Set enthaltend eine pulverförmige Zusammensetzung gemäß einer der Ausführungsformen 1 bis 22 und einen nasalen Applikator wie einen Schnupflöffel oder ein Schnupfröhrchen.
Ausführungsform 28. Set gemäß Ausführungsform 27, dadurch gekennzeichnet, dass das Set ein Behältnis gemäß einer der Ausführungsformen 23 bis 26 enthält.
Ausführungsform 29: Set gemäß Ausführungsform 27 oder 28, dadurch gekennzeichnet, dass das Set mindestens zwei, vorzugsweise mindestens drei, insbesondere mindestens fünf Behältnisse gemäß einer der Ausführungsformen 23 bis 26 enthält.
Ausführungsform 30: Verwendung einer pulverförmige Zusammensetzung gemäß einer der Ausführungsformen 1 bis 22, einem Behältnis gemäß einer der Ausführungsformen 23 bis 26, oder einem Set gemäß einer der Ausführungsformen 27 bis 29 zur nasalen Applikation der pulverförmigen Zusammensetzung.
Ausführungsform 31: Verwendung gemäß Ausführungsform 30, dadurch gekennzeichnet, dass die Verwendung die nasale Applikation von mindestens 10 mg, bevorzugt mindestens 50 mg, mehr bevorzugt mindestens 200 mg, noch mehr bevorzugt mindestens 400 mg, am meisten bevorzugt mindestens 800 mg der pulverförmigen Zusammensetzung umfasst.
Ausführungsform 32: Verwendung gemäß Ausführungsform 30 oder 31, dadurch gekennzeichnet, dass die Verwendung die nasale Applikation von zwischen 10 mg und 5.000 mg, bevorzugt zwischen 50 mg und 4.000 mg, mehr bevorzugt zwischen 200 mg und 3.000 mg, noch mehr bevorzugt zwischen 400 mg und 2.000 mg, am meisten bevorzugt zwischen 800 mg und 1.250 mg der pulverförmigen Zusammensetzung umfasst.
Ausführungsform 33: Verwendung einer pulverförmigen Zusammensetzung enthaltend Taurin, Koffein, L-Arginin und L-Tryptophan zur Verbesserung der kognitiven Leistungsfähigkeit, insbesondere zur Steigerung der Konzentrationsfähigkeit und/oder der neuralen Effizienz, wobei die pulverförmige Zusammensetzung nasal appliziert wird, vorzugsweise wobei die Zusammensetzung wie in einer der Ausführungsformen 2 bis 22 definiert ist.
Ausführungsform 34: Verwendung einer pulverförmigen Zusammensetzung enthaltend Taurin, Koffein, L-Arginin und L-Tryptophan zur Verhinderung einer Überaktivierung von Hirnregionen bei Stressbelastung und/oder zur Verbesserung der Downregulierung nach einer Stressbelastung, wobei die pulverförmige Zusammensetzung nasal appliziert wird, vorzugsweise wobei die Zusammensetzung wie in einer der Ausführungsformen 2 bis 22 definiert ist.
Ausführungsform 35: Verwendung einer pulverförmigen Zusammensetzung enthaltend Taurin, Koffein, L-Arginin und L-Tryptophan zur Verbesserung der körperlichen Leistungsfähigkeit, insbesondere zur Steigerung der Kraft und/oder der Ausdauer, wobei die pulverförmige Zusammensetzung nasal appliziert wird, vorzugsweise wobei die Zusammensetzung wie in einer der Ausführungsformen 2 bis 22 definiert ist.
Ausführungsform 36: Verwendung einer pulverförmigen Zusammensetzung enthaltend Taurin, Koffein, L-Arginin und L-Tryptophan zur Stimmungsaufhellung, wobei die pulverförmige Zusammensetzung nasal appliziert wird, vorzugsweise wobei die Zusammensetzung wie in einer der Ausführungsformen 2 bis 22 definiert ist.
Ausführungsform 37: Verwendung einer pulverförmigen Zusammensetzung enthaltend Taurin, Koffein, L-Arginin und L-Tryptophan zur Anregung der Gehirnaktivität, wobei die pulverförmige Zusammensetzung nasal appliziert wird, vorzugsweise wobei die Zusammensetzung wie in einer der Ausführungsformen 2 bis 22 definiert ist.
Ausführungsform 38: Verwendung einer pulverförmigen Zusammensetzung enthaltend Taurin, Koffein, L-Arginin und L-Tryptophan als Aroma, wobei die pulverförmige Zusammensetzung nasal appliziert wird, vorzugsweise wobei die Zusammensetzung wie in einer der Ausführungsformen 2 bis 22 definiert ist.
Ausführungsform 39: Verwendung einer pulverförmigen Zusammensetzung enthaltend Taurin, Koffein, L-Arginin und L-Tryptophan zur Verstärkung der Wirkung von Aromen, wobei die pulverförmige Zusammensetzung nasal appliziert wird, vorzugsweise wobei die Zusammensetzung wie in einer der Ausführungsformen 2 bis 22 definiert ist.
Ausführungsform 40: Verwendung gemäß einer der Ausführungsformen 30 bis 39, wobei die Tagesdosis der pulverförmigen Zusammensetzung zwischen 10 mg und 20.000 mg, bevorzugt zwischen 50 mg und 10.000 mg, mehr bevorzugt zwischen 200 mg und 5.000 mg, noch mehr bevorzugt zwischen 400 mg und 2.500 mg, am meisten bevorzugt zwischen 800 mg und 1.250 mg beträgt.

Die Erfindung wird auch anhand des folgenden Beispiels, auf das sie selbstverständlich nicht eingeschränkt ist, näher erläutert.

### Beispiel:

1 kg der pulverförmigen Zusammensetzung wird durch Mischung der folgenden Bestandteile (in Pulverform) erhalten: 250 g Taurin, 150 g L-Arginin HCl, 100 g Koffein anhydrat, 100 g L-Tryptophan und 400 g Maltodextrin. Die mittlere Partikelgröße der Zusammensetzung beträgt 75 µm. Die Zusammensetzung wird in Sachets zu je 1 Gramm abgefüllt.

Mittels Schnupflöffel werden 5x 200 mg (d.h. der Gesamtinhalt eines Sachets) über den Tag verteilt an einen erwachsenen menschlichen Probanden verabreicht.

## Patentansprüche

1. Pulverförmige Zusammensetzung zur nasalen Applikation enthaltend Taurin, Koffein, L-Arginin und L-Tryptophan.

2. Pulverförmige Zusammensetzung gemäß Anspruch 1, **dadurch gekennzeichnet, dass** die Zusammensetzung eine mittlere Partikelgröße von mehr als 1 µm, bevorzugt zwischen 1 µm und 1.000 µm, aufweist.

3. Pulverförmige Zusammensetzung gemäß Anspruch 1 oder 2, **dadurch gekennzeichnet, dass** die Zusammensetzung enthält:
- zwischen 4 Gew.-% und 40 Gew.-% Taurin;
- zwischen 2 Gew.-% und 25 Gew.-% Koffein;
- zwischen 1 Gew.-% und 17 Gew.-% L-Arginin; und
- zwischen 1 Gew.-% und 17 Gew.-% L-Tryptophan.

4. Behältnis enthaltend eine pulverförmige Zusammensetzung gemäß einem der Ansprüche 1 bis 3.

5. Behältnis gemäß Anspruch 4, **dadurch gekennzeichnet, dass** das Behältnis mindestens 10 mg, bevorzugt zwischen 10 mg und 20.000 mg, der pulverförmigen Zusammensetzung enthält.

6. Set enthaltend eine pulverförmige Zusammensetzung gemäß einem der Ansprüche 1 bis 3 und einen nasalen Applikator wie einen Schnupflöffel oder ein Schnupfröhrchen.

7. Set gemäß Anspruch 6, **dadurch gekennzeichnet, dass** das Set mindestens ein, vorzugsweise mindestens zwei, mehr bevorzugt mindestens drei, insbesondere mindestens fünf Behältnisse gemäß Anspruch 4 oder 5 enthält.

8. Verwendung einer pulverförmige Zusammensetzung gemäß einem der Ansprüche 1 bis 3, einem Behältnis gemäß Anspruch 4 oder 5, oder einem Set gemäß Anspruch 6 oder 7 zur nasalen Applikation der pulverförmigen Zusammensetzung.

9. Verwendung einer pulverförmigen Zusammensetzung enthaltend Taurin, Koffein, L-Arginin und L-Tryptophan zur Verbesserung der kognitiven Leistungsfähigkeit, insbesondere zur Steigerung der Konzentrationsfähigkeit und/oder der neuralen Effizienz, wobei die pulverförmige Zusammensetzung nasal appliziert wird, vorzugsweise wobei die Zusammensetzung wie in Anspruch 2 oder 3 definiert ist.

10. Verwendung einer pulverförmigen Zusammensetzung enthaltend Taurin, Koffein, L-Arginin und L-Tryptophan zur Verhinderung einer Überaktivierung von Hirnregionen bei Stressbelastung und/oder zur Verbesserung der Downregulierung nach einer Stressbelastung, wobei die pulverförmige Zusammensetzung nasal appliziert wird, vorzugsweise wobei die Zusammensetzung wie in Anspruch 2 oder 3 definiert ist.

11. Verwendung einer pulverförmigen Zusammensetzung enthaltend Taurin, Koffein, L-Arginin und L-Tryptophan zur Verbesserung der körperlichen Leistungsfähigkeit, insbesondere zur Steigerung der Kraft und/oder der Ausdauer, wobei die pulverförmige Zusammensetzung nasal appliziert wird, vorzugsweise wobei die Zusammensetzung wie in Anspruch 2 oder 3 definiert ist.

12. Verwendung einer pulverförmigen Zusammensetzung enthaltend Taurin, Koffein, L-Arginin und L-Tryptophan zur Stimmungsaufhellung, wobei die pulverförmige Zusammensetzung nasal appliziert wird, vorzugsweise wobei die Zusammensetzung wie in Anspruch 2 oder 3 definiert ist.

13. Verwendung einer pulverförmigen Zusammensetzung enthaltend Taurin, Koffein, L-Arginin und L-Tryptophan zur Anregung der Gehirnaktivität, wobei die pulverförmige Zusammensetzung nasal appliziert wird, vorzugsweise wobei die Zusammensetzung wie in Anspruch 2 oder 3 definiert ist.

14. Verwendung einer pulverförmigen Zusammensetzung enthaltend Taurin, Koffein, L-Arginin und L-Tryptophan als Aroma, wobei die pulverförmige Zusammensetzung nasal appliziert wird, vorzugsweise wobei die Zusammensetzung wie in Anspruch 2 oder 3 definiert ist.

15. Verwendung einer pulverförmigen Zusammensetzung enthaltend Taurin, Koffein, L-Arginin und L-Tryptophan zur Verstärkung der Wirkung von Aromen, wobei die pulverförmige Zusammensetzung nasal appliziert wird, vorzugsweise wobei die Zusammensetzung wie in Anspruch 2 oder 3 definiert ist.
